# EUROPEAN PATENT APPLICATION

(11) **EP 4 843 494 A1**
(43) Date of publication of application: **25.11.2026**
(21) Application number: 25741447.4
(22) Date of filing: 13.01.2025
(51) Int. Cl.: A61B 5/1455, A61B 5/0205

(54) **DATA PROCESSING METHOD, DATA PROCESSING APPARATUS, ELECTRONIC DEVICE, AND READABLE STORAGE MEDIUM**

(30) Priority: 17.01.2024 CN 202410069116
(71) Applicant: Vivo Mobile Communication Co., Ltd., Dongguan, Guangdong 523863 (CN)
(72) Inventor: LI, Fangguo, Dongguan, Guangdong 523863 (CN)
(74) Representative: Conti, Marco
(86) International application number: PCT/CN2025/071991
(87) International publication number: WO 2025/152885

(57) **Abstract**

This application discloses a data processing method, a data processing apparatus, an electronic device, and a readable storage medium, and belongs to the field of blood oxygen measurement technologies. The data processing method includes: obtaining a health parameter, where the health parameter includes a photoplethysmography signal; determining a green light amplitude spectrum, a red light amplitude spectrum, and an infrared light amplitude spectrum based on the photoplethysmography signal; determining a human pulse frequency based on the green light amplitude spectrum; determining a first parameter based on the red light amplitude spectrum and the human pulse frequency, and determining a second parameter based on the infrared light amplitude spectrum and the human pulse frequency; and determining blood oxygen saturation based on the first parameter and the second parameter.

## Description

### CROSS-REFERENCE TO RELATED APPLICATIONS

This application claims priority to Chinese Patent Application No. 202410069116.4, entitled "DATA PROCESSING METHOD, DATA PROCESSING APPARATUS, ELECTRONIC DEVICE, AND READABLE STORAGE MEDIUM" and filed on January 17, 2024, which is incorporated herein by reference in its entirety.

### TECHNICAL FIELD

This application belongs to the field of blood oxygen measurement technologies, and specifically, relates to a data processing method, a data processing apparatus, an electronic device, and a readable storage medium.

### BACKGROUND

Blood oxygen saturation (SaO2) is a percentage of a concentration of oxyhemoglobin (HbO2) to a concentration of all functional hemoglobin (Hb, hemoglobin) in blood, that is, a blood oxygen concentration in blood. The blood oxygen saturation is a vital physiological parameter of a respiratory and circulatory system. When deoxyhemoglobin in blood combines with oxygen entering the human body, it forms oxyhemoglobin. The deoxyhemoglobin and the oxyhemoglobin have different absorption ratios for infrared light and red light. The oxyhemoglobin absorbs less red light and more infrared light; while the deoxyhemoglobin absorbs more red light and less infrared light. Generally, wearable devices having a blood oxygen measurement function use this characteristic to calculate the blood oxygen saturation.

In the related art, wearable smart devices, such as smart watches, determine, based on only red light and infrared light, a parameter for calculating the blood oxygen saturation. In some application scenarios, reflected signals of the red light and the infrared light are characterized by low perfusion indexes and weak signal strength, resulting in low accuracy of calculated blood oxygen saturation.

### SUMMARY

An objective of embodiments of this application is to provide a data processing method, a data processing apparatus, an electronic device, and a readable storage medium, which can resolve a problem of low accuracy of blood oxygen saturation determined only through red light and infrared light.

According to a first aspect, the embodiments of this application provide a data processing method. The data processing method includes: obtaining a health parameter, where the health parameter includes a photoplethysmography signal; determining a green light amplitude spectrum, a red light amplitude spectrum, and an infrared light amplitude spectrum based on the photoplethysmography signal; determining a human pulse frequency based on the green light amplitude spectrum; determining a first parameter based on the red light amplitude spectrum and the human pulse frequency, and determining a second parameter based on the infrared light amplitude spectrum and the human pulse frequency; and determining blood oxygen saturation based on the first parameter and the second parameter.

According to a second aspect, the embodiments of this application provide a data processing apparatus. The data processing apparatus includes: a processing unit, configured to determine a first parameter and a second parameter, and determine blood oxygen saturation based on the first parameter and the second parameter.

According to a third aspect, the embodiments of this application provide an electronic device. The electronic device includes a processor and a memory, where the memory stores a program or instructions executable on the processor, and when the program or the instructions are executed by the processor, the steps of the data processing method according to the first aspect are implemented.

According to a fourth aspect, the embodiments of this application provide a readable storage medium, storing a program or instructions executable on a processor, where the program or the instructions, when executed by the processor, implement the steps of the data processing method according to the first aspect.

According to a fifth aspect, the embodiments of this application provide a chip, including a processor and a communication interface, where the communication interface is coupled to the processor, and the processor is configured to execute a program or instructions to implement the steps of the data processing method according to the first aspect.

According to a sixth aspect, the embodiments of this application provide a computer program product, where the program product is stored in a storage medium, and the program product is executed by at least one processor to implement the steps of the data processing method according to the first aspect.

According to a seventh aspect, the embodiments of this application provide an electronic device, configured to perform the steps of the data processing method according to the first aspect.

A data processing method provided in the embodiments of this application includes: obtaining a health parameter, where the health parameter includes a photoplethysmography signal; determining a green light amplitude spectrum, a red light amplitude spectrum, and an infrared light amplitude spectrum based on the photoplethysmography signal; determining a human pulse frequency based on the green light amplitude spectrum; determining a first parameter based on the red light amplitude spectrum and the human pulse frequency, and determining a second parameter based on the infrared light amplitude spectrum and the human pulse frequency; and determining blood oxygen saturation based on the first parameter and the second parameter. In the embodiments of this application, the human pulse frequency is extracted through green light with a higher signal-to-noise ratio (the human pulse frequency is determined based on the green light amplitude spectrum), and the first parameter and the second parameter are determined based on the human pulse frequency. This approach to determining whether conditions for calculating the blood oxygen saturation are met and this approach to determining the parameters used for calculating the blood oxygen saturation lead to easier signal feature extraction and higher reliability of obtained first and second parameters, so that the finally calculated blood oxygen saturation can be more accurate.

### BRIEF DESCRIPTION OF THE DRAWINGS

FIG. 1 is a flowchart 1 of a data processing method according to an embodiment of this application;
FIG. 2 is a flowchart 2 of a data processing method according to an embodiment of this application;
FIG. 3 is a flowchart 3 of a data processing method according to an embodiment of this application;
FIG. 4 is a flowchart 4 of a data processing method according to an embodiment of this application;
FIG. 5 is a structural block diagram of a data processing apparatus according to an embodiment of this application;
FIG. 6 is a structural block diagram of an electronic device according to an embodiment of this application;
FIG. 7 is a schematic structural diagram of hardware of an electronic device according to an embodiment of this application;
FIG. 8 is a schematic diagram of a photoplethysmography signal with a direct current component filtered out according to an embodiment of this application;
FIG. 9 is a schematic diagram of a green light amplitude spectrum, a red light amplitude spectrum, and an infrared light amplitude spectrum according to an embodiment of this application;
FIG. 10 is a schematic diagram of a green light amplitude spectrum according to an embodiment of this application;
FIG. 11 is a schematic diagram of a red light amplitude spectrum according to an embodiment of this application;
FIG. 12 is a schematic diagram 1 of an infrared light amplitude spectrum according to an embodiment of this application; and
FIG. 13 is a schematic diagram 2 of an infrared light amplitude spectrum according to an embodiment of this application.

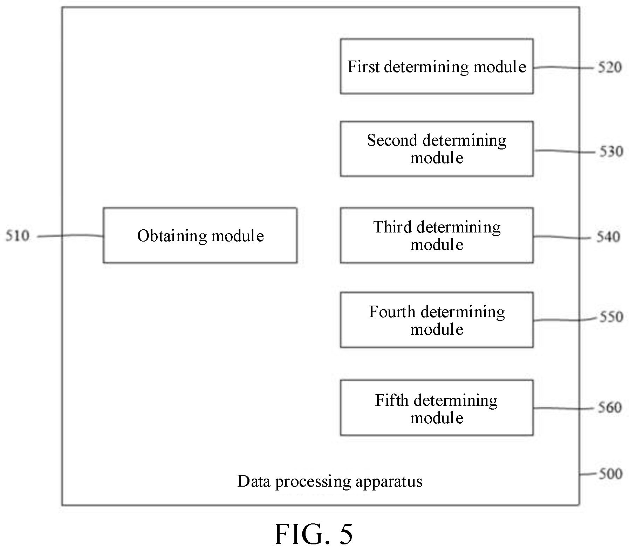

A correspondence between reference numerals and component names in FIG. 5 is as follows:
500: data processing apparatus; 510: obtaining module; 520: first determining module; 530: second determining module; 540: third determining module; 550: fourth determining module; and 560: fifth determining module.

### DETAILED DESCRIPTION

The following clearly describes technical solutions in the embodiments of this application with reference to the accompanying drawings in the embodiments of this application. Apparently, the described embodiments are some of the embodiments of this application rather than all of the embodiments. All other embodiments derived by a person of ordinary skill in the art based on the embodiments of this application shall fall within the protection scope of this application.

In the specification and claims of this application, the terms "first", "second", and the like are used to distinguish similar objects, but are not used to describe a specific sequence or order. It should be understood that the terms used in such a way are interchangeable in proper circumstances, so that the embodiments of this application can be implemented in other sequences than the sequences illustrated or described herein, and the objects distinguished through "first", "second", and the like are generally of a same type and a quantity of objects are not limited, for example, a first object may be one or more than one. In addition, "and/or" in this specification and the claims represents at least one of the connected objects, and a character "/" used herein generally indicates an "or" relationship between associated objects.

A data processing method, a data processing apparatus, an electronic device, and a readable storage medium according to the embodiments of this application are described in detail below with reference to the accompanying drawings through specific embodiments and application scenarios thereof.

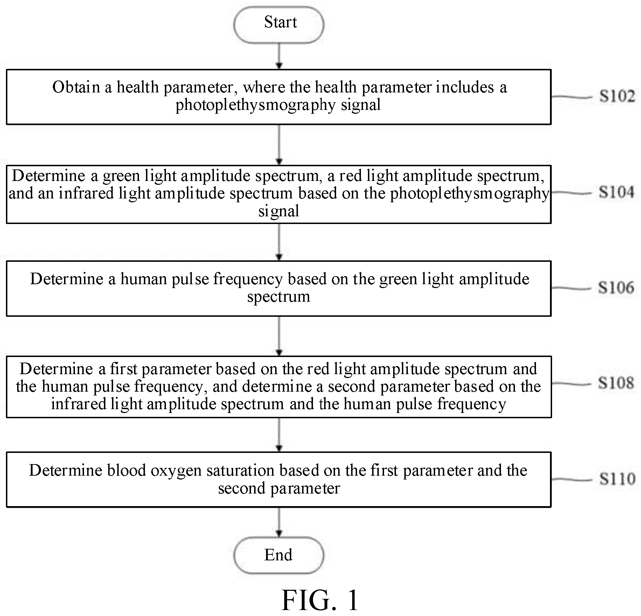

As shown in FIG. 1, FIG. 2, and FIG. 3, an embodiment of this application provides a data processing method. It should be noted that the data processing method may be implemented through a wearable device, or certainly may be implemented through a device of another type, such as a mobile phone. The wearable device is a smart watch, a smart bracelet, or another smart device that a user can wear.

In an embodiment according to this application, as shown in FIG. 1, the data processing method includes the following specific steps.

S102: Obtain a health parameter, where the health parameter includes a photoplethysmography signal.

The photoplethysmography signal (PPG signal) not only includes a red light signal and an infrared light signal, but also includes a green light signal. It is determined, based on the green light signal, the red light signal, and the infrared light signal, whether conditions for calculating blood oxygen saturation are met. When the conditions for calculating the blood oxygen saturation are met, some parameters required for calculating the blood oxygen saturation are determined based on the green light signal, the red light signal, and the infrared light signal.

S104: Determine a green light amplitude spectrum, a red light amplitude spectrum, and an infrared light amplitude spectrum based on the photoplethysmography signal.

Optionally, the photoplethysmography signal is processed through a first filter, and a red light direct current amplitude and an infrared light direct current amplitude are extracted from the photoplethysmography signal. An objective of this step is to pre-process the photoplethysmography signal. A frequency of a human photoplethysmography signal generally ranges from 0.5 Hz to 5 Hz. Considering that there is a harmonic in the photoplethysmography signal and that a harmonic above a third harmonic is less significant, the photoplethysmography signal is processed through the first filter in this step. Optionally, the first filter is a low-pass filter. The low-pass filter is an electronic filtering apparatus that allows a signal below a cut-off frequency to pass through but blocks a signal above the cut-off frequency. Optionally, the cut-off frequency of the low-pass filter ranges from 14 Hz to 16 Hz. Optionally, the cut-off frequency of the low-pass filter is 15 Hz. This step is used for determining the red light direct current amplitude (DC_{red}) and the infrared light direct current amplitude (DCᵢᵣ) as parameters for calculating the blood oxygen saturation in subsequent steps.

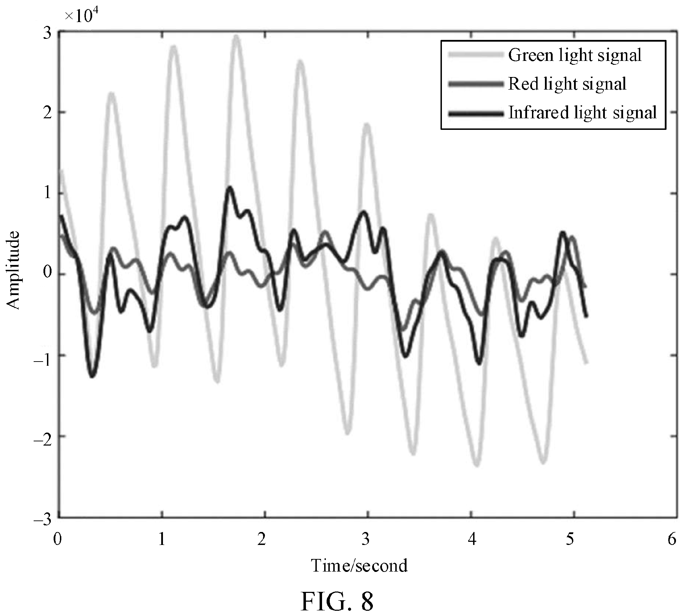

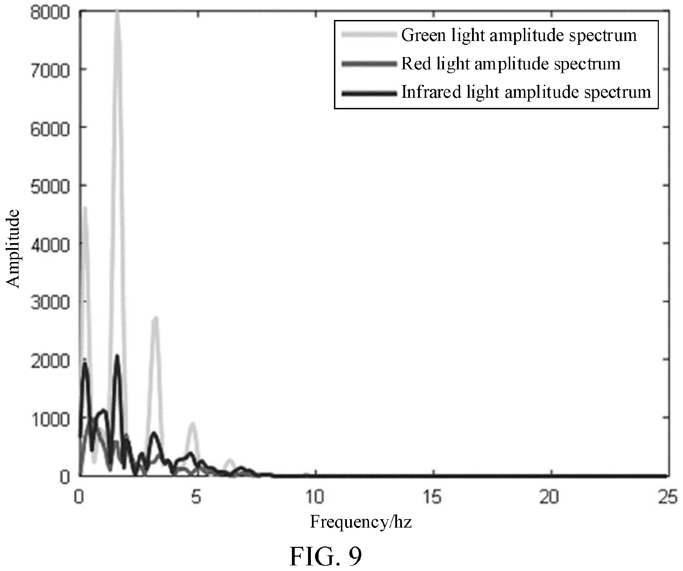

Optionally, a direct current component in the photoplethysmography signal is removed through a second filter, and the green light amplitude spectrum, the red light amplitude spectrum, and the infrared light amplitude spectrum are determined. Optionally, the second filter is a high-pass filter. The high-pass filter is an electronic filtering apparatus that allows a signal above a cut-off frequency to pass through but blocks a signal below the cut-off frequency. Optionally, the cut-off frequency of the high-pass filter ranges from 0.4 Hz to 0.6 Hz. The cut-off frequency of the high-pass filter is 0.5 Hz. FIG. 8 is a schematic diagram of a photoplethysmography signal with a direct current component filtered out according to an embodiment of this application. After the direct current component in the photoplethysmography signal is removed through the second filter, the green light amplitude spectrum is determined through Fourier transform based on the green light signal; the red light amplitude spectrum is determined through Fourier transform based on the red light signal; and the infrared light amplitude spectrum is determined through Fourier transform based on the infrared light signal. FIG. 9 is a schematic diagram of a green light amplitude spectrum, a red light amplitude spectrum, and an infrared light amplitude spectrum according to an embodiment of this application. It should be noted that the Fourier transform belongs to harmonic analysis. In signal processing, a purpose of the Fourier transform is to decompose a signal into a frequency spectrum (an amplitude spectrum). The frequency spectrum is used to show a magnitude of an amplitude corresponding to a frequency.

S106: Determine a human pulse frequency based on the green light amplitude spectrum.

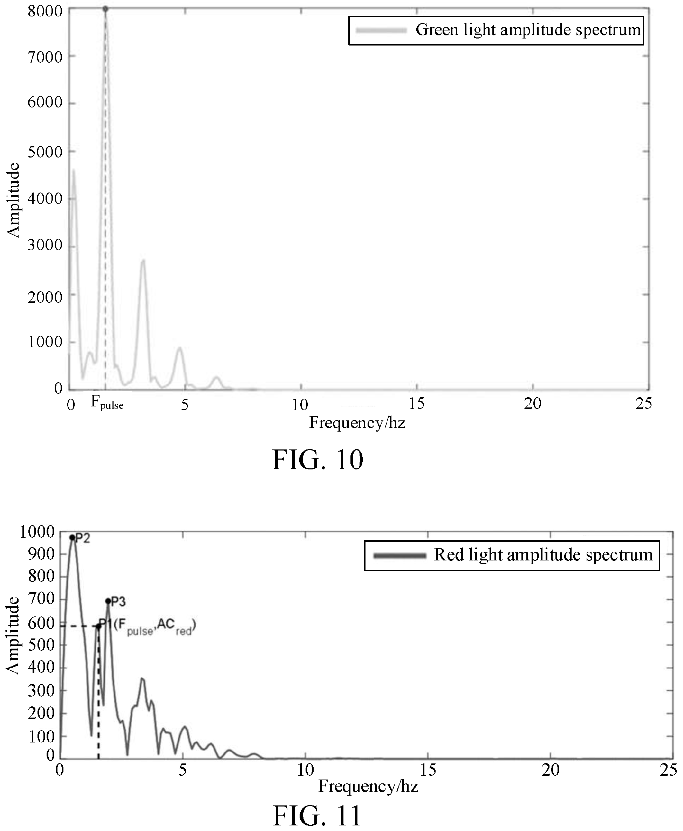

Compared with red light and infrared light, green light is more likely to be absorbed by oxyhemoglobin and deoxyhemoglobin. Compared with the green light, the red light and the infrared light penetrate skin tissue more easily. Therefore, the green light has a larger signal variation amplitude and a higher signal-to-noise ratio in a reflective blood oxygen measurement apparatus. The signal-to-noise ratio (signal to interference plus noise ratio, Signal to Interference plus Noise Ratio) refers to a ratio of strength of a received desired signal to strength of a received interference signal (noise and interference). Although signal-to-noise ratios of photoplethysmography signals collected through light of different wavelengths are different, pulse frequencies thereof are the same. In other words, the green light with the higher signal-to-noise ratio can be used for extracting a pulse frequency (the human pulse frequency), the human pulse frequency can be more easily determined based on the green light amplitude spectrum, leading to a more accurate measurement result. Optionally, as shown in FIG. 10, a frequency corresponding to a maximum amplitude in the green light amplitude spectrum is used as the human pulse frequency Fₚᵤₗₛₑ.

S108: Determine a first parameter based on the red light amplitude spectrum and the human pulse frequency, and determine a second parameter based on the infrared light amplitude spectrum and the human pulse frequency.

Optionally, the first parameter includes a red light signal-to-noise ratio, a red light alternating current amplitude, and a red light second harmonic amplitude.

Optionally, the second parameter includes an infrared light signal-to-noise ratio, an infrared light alternating current amplitude, and an infrared light second harmonic amplitude.

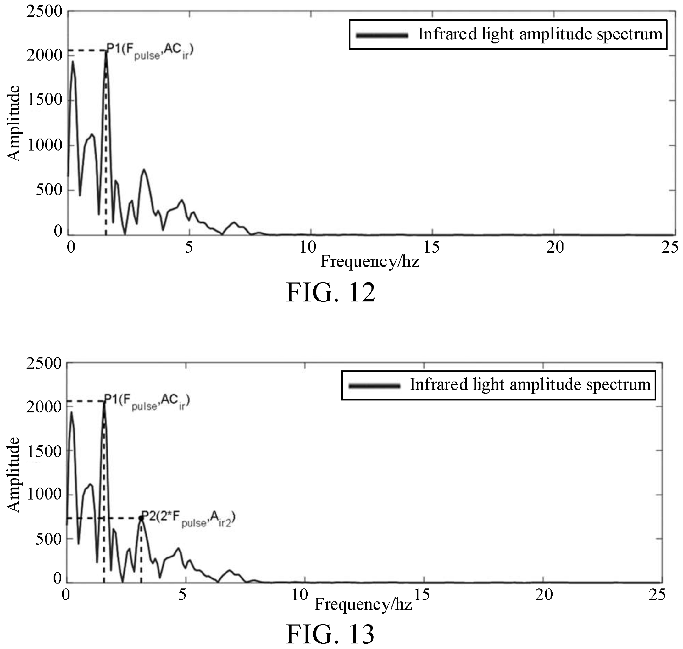

Optionally, a red light signal-to-noise ratio SNR_{red} is determined based on the red light amplitude spectrum and the human pulse frequency; and an infrared light signal-to-noise ratio SNRᵢᵣ is determined based on the infrared light amplitude spectrum and the human pulse frequency. Optionally, as shown in FIG. 11, a red light alternating current amplitude AC_{red} can also be determined based on the red light amplitude spectrum and the human pulse frequency. The red light alternating current amplitude is an amplitude corresponding to the human pulse frequency in the red light amplitude spectrum. An amplitude corresponding to P1 in FIG. 11 is the correct red light alternating current amplitude AC_{red}. Both an amplitude corresponding to P2 and an amplitude corresponding to P3 in FIG. 11 are greater than the amplitude corresponding to P1. If no human pulse frequency is found by using the green light amplitude spectrum, the amplitude corresponding to P2 and the amplitude corresponding to P3 are easily misidentified as the red light alternating current amplitude. Optionally, as shown in FIG. 12, an infrared light alternating current amplitude ACᵢᵣ can also be determined based on the infrared light amplitude spectrum and the human pulse frequency. The infrared light alternating current amplitude is an amplitude corresponding to the human pulse frequency in the infrared light amplitude spectrum. An amplitude corresponding to P1 in FIG. 12 is the correct infrared light alternating current amplitude ACᵢᵣ. Optionally, a second harmonic frequency is determined based on the human pulse frequency. Twice the human pulse frequency is used as the second harmonic frequency. Optionally, a red light second harmonic amplitude A_{red2} is determined based on the red light amplitude spectrum and the second harmonic frequency. The red light second harmonic amplitude is an amplitude corresponding to the second harmonic frequency in the red light amplitude spectrum. Optionally, as shown in FIG. 13, an infrared light second harmonic amplitude Aᵢᵣ₂ is determined based on the infrared light amplitude spectrum and the second harmonic frequency. The infrared light second harmonic amplitude is an amplitude corresponding to the second harmonic frequency in the infrared light amplitude spectrum. An amplitude corresponding to P1 in FIG. 13 is the correct infrared light alternating current amplitude ACᵢᵣ. An amplitude corresponding to P2 in FIG. 13 is the correct infrared light second harmonic amplitude Aᵢᵣ₂. Optionally, a red light harmonic amplitude ratio Harmonic_ratio_{red} is determined based on the red light second harmonic amplitude and the red light alternating current amplitude. Optionally, an infrared light harmonic amplitude ratio Harmonic_ratioᵢᵣ is determined based on the infrared light second harmonic amplitude and the infrared light alternating current amplitude. Because the human photoplethysmography signal generally has a significant second harmonic, and it is usually difficult for interference to form a second harmonic, the presence of a significant second harmonic in a signal spectrum can be used to determine whether the photoplethysmography signal is a physiological signal. Considering that signal strength is affected by factors such as a light emission current of a sensor, when a signal is weak due to a small light emission current, if whether the signal is a physiological signal is determined by directly using a magnitude of a second harmonic amplitude, the weak signal is prone to being incorrectly determined as a non-physiological signal. In this case, it is more appropriate to determine, by using a ratio of a second harmonic amplitude to an alternating current amplitude, whether a signal is a physiological signal. When both a signal-to-noise ratio and a harmonic amplitude meet a blood oxygen measurement condition, the blood oxygen saturation is further calculated.

S110: Determine the blood oxygen saturation based on the first parameter and the second parameter.

It is determined, based on the red light signal-to-noise ratio, the infrared light signal-to-noise ratio, the red light harmonic amplitude ratio, and the infrared light harmonic amplitude ratio, whether to calculate the blood oxygen saturation. The blood oxygen saturation is determined based on the red light alternating current amplitude, the red light direct current amplitude, the infrared light alternating current amplitude, and the infrared light direct current amplitude when it is determined that the photoplethysmography signal is a physiological signal and suitable for calculating the blood oxygen saturation. Compared with an approach to determining quality of the photoplethysmography signal based on a correlation coefficient between an alternating current part in the red light signal and an alternating current part in the green light signal or a correlation coefficient between an alternating current part in the infrared light signal and an alternating current part in the green light signal, this approach to determining, based on a combination of the signal-to-noise ratio and the harmonic amplitude ratio, whether the photoplethysmography signal is a physiological signal and whether the photoplethysmography signal is suitable for calculating the blood oxygen saturation is not prone to incorrectly determining a non-physiological signal as a physiological signal and is not prone to incorrectly determining a weak signal as a non-physiological signal, leading to a more accurate determining result.

In the embodiments of this application, the human pulse frequency is extracted through green light with a higher signal-to-noise ratio (the human pulse frequency is determined based on the green light amplitude spectrum), and the first parameter and the second parameter are determined based on the human pulse frequency. This approach to determining whether conditions for calculating the blood oxygen saturation are met and this approach to determining the parameters used for calculating the blood oxygen saturation lead to easier signal feature extraction and higher reliability of obtained first and second parameters, so that the finally calculated blood oxygen saturation can be more accurate.

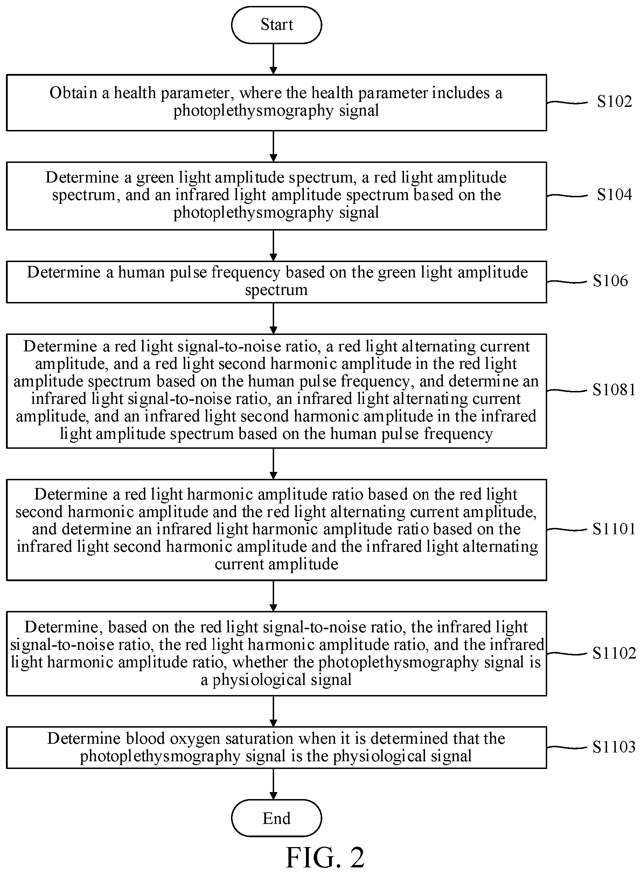

In another embodiment of this application, as shown in FIG. 2, step S108 specifically includes the following step.

S1081: Determine a red light signal-to-noise ratio, a red light alternating current amplitude, and a red light second harmonic amplitude in the red light amplitude spectrum based on the human pulse frequency, and determine an infrared light signal-to-noise ratio, an infrared light alternating current amplitude, and an infrared light second harmonic amplitude in the infrared light amplitude spectrum based on the human pulse frequency.

Optionally, a red light signal-to-noise ratio SNR_{red} is determined based on the red light amplitude spectrum and the human pulse frequency; and an infrared light signal-to-noise ratio SNRᵢᵣ is determined based on the infrared light amplitude spectrum and the human pulse frequency. Optionally, the red light alternating current amplitude, the red light second harmonic amplitude, the infrared light alternating current amplitude, and the infrared light second harmonic amplitude are determined based on the human pulse frequency. As shown in FIG. 11, a red light alternating current amplitude AC_{red} can also be determined based on the red light amplitude spectrum and the human pulse frequency. The red light alternating current amplitude is an amplitude corresponding to the human pulse frequency in the red light amplitude spectrum. An amplitude corresponding to P1 in FIG. 11 is the correct red light alternating current amplitude AC_{red}. Both an amplitude corresponding to P2 and an amplitude corresponding to P3 in FIG. 11 are greater than the amplitude corresponding to P1. If the human pulse frequency is not determined by using the green light amplitude spectrum, the amplitude corresponding to P2 and the amplitude corresponding to P3 are easily misidentified as the red light alternating current amplitude. Optionally, as shown in FIG. 12, an infrared light alternating current amplitude ACᵢᵣ can also be determined based on the infrared light amplitude spectrum and the human pulse frequency. The infrared light alternating current amplitude is an amplitude corresponding to the human pulse frequency in the infrared light amplitude spectrum. An amplitude corresponding to P1 in FIG. 12 is the correct infrared light alternating current amplitude ACᵢᵣ. Optionally, a second harmonic frequency is determined based on the human pulse frequency. Twice the human pulse frequency is used as the second harmonic frequency. Optionally, a red light second harmonic amplitude A_{red2} is determined based on the red light amplitude spectrum and the second harmonic frequency. The red light second harmonic amplitude is an amplitude corresponding to the second harmonic frequency in the red light amplitude spectrum. Optionally, as shown in FIG. 13, an infrared light second harmonic amplitude Aᵢᵣ₂ is determined based on the infrared light amplitude spectrum and the second harmonic frequency. The infrared light second harmonic amplitude is an amplitude corresponding to the second harmonic frequency in the infrared light amplitude spectrum. An amplitude corresponding to P1 in FIG. 13 is the correct infrared light alternating current amplitude ACᵢᵣ. An amplitude corresponding to P2 in FIG. 13 is the correct infrared light second harmonic amplitude Aᵢᵣ₂.

In another embodiment of this application, as shown in FIG. 2, step S110 includes the following steps.

S1101: Determine a red light harmonic amplitude ratio based on the red light second harmonic amplitude and the red light alternating current amplitude, and determine an infrared light harmonic amplitude ratio based on the infrared light second harmonic amplitude and the infrared light alternating current amplitude.

Optionally, an infrared light second harmonic amplitude Aᵢᵣ₂ is determined based on the infrared light amplitude spectrum and the second harmonic frequency. The infrared light second harmonic amplitude is an amplitude corresponding to the second harmonic frequency in the infrared light amplitude spectrum. A red light harmonic amplitude ratio Harmonic_ratio_{red} is a ratio of the red light second harmonic amplitude to the red light alternating current amplitude. An infrared light harmonic amplitude ratio Harmonic_ratioᵢᵣ is a ratio of the infrared light second harmonic amplitude to the infrared light alternating current amplitude.

S1102: Determine, based on the red light signal-to-noise ratio, the infrared light signal-to-noise ratio, the red light harmonic amplitude ratio, and the infrared light harmonic amplitude ratio, whether the photoplethysmography signal is a physiological signal.

When both the red light signal-to-noise ratio and the infrared light signal-to-noise ratio meet a threshold requirement, it indicates that the signal meets the blood oxygen measurement condition. When the red light signal-to-noise ratio or the infrared light signal-to-noise ratio does not meet the threshold requirement, it is considered that the signal does not meet the blood oxygen measurement condition. In addition, because the human photoplethysmography signal generally has a significant second harmonic, and it is usually difficult for interference to form a second harmonic, the presence of a significant second harmonic in a signal spectrum can be used to determine whether the photoplethysmography signal is a physiological signal. Considering that signal strength is affected by factors such as a light emission current of a sensor, when a signal is weak due to a small light emission current, if the signal strength is determined by directly using a magnitude of a second harmonic amplitude, the weak signal is prone to being incorrectly determined as a non-physiological signal. In this case, it is more appropriate to determine, by using a ratio of a second harmonic amplitude to an alternating current amplitude, whether a signal is a physiological signal. When both a signal-to-noise ratio and a harmonic amplitude meet a blood oxygen measurement condition, the blood oxygen saturation is further calculated.

S1103: Determine the blood oxygen saturation when it is determined that the photoplethysmography signal is the physiological signal.

Optionally, the blood oxygen saturation is determined based on the red light alternating current amplitude, the red light direct current amplitude, the infrared light alternating current amplitude, and the infrared light direct current amplitude.

Optionally, a first ratio is determined based on the red light alternating current amplitude, the red light direct current amplitude, the infrared light alternating current amplitude, and the infrared light direct current amplitude; and the blood oxygen saturation is determined based on the first ratio.

In some embodiments, optionally, the first ratio is a ratio of a third ratio to a fourth ratio. The third ratio is a ratio of the red light alternating current amplitude to the red light direct current amplitude (AC_{red}/DC_{red}). The fourth ratio is a ratio of the infrared light alternating current amplitude to the infrared light direct current amplitude (ACᵢᵣ/DCᵢᵣ). The first ratio R=(AC_{red}/DC_{red})/(ACᵢᵣ/DCᵢᵣ).

In some embodiments, optionally, the blood oxygen saturation and the first ratio satisfy a first formula. The first formula is: SₚO₂=A+B×R+C×R². SₚO₂ is the blood oxygen saturation, R is the first ratio, and A, B, and C are constants.

It should be noted that the first formula is determined through a desaturation study. During the experiment, data is collected to calculate the ratio R (the first ratio), a blood gas analyzer is used to obtain blood oxygen saturation at a corresponding moment, and the blood oxygen saturation is used as a gold-standard blood oxygen result. A functional relationship is determined by fitting several R values and gold-standard blood oxygen results, to obtain the first formula.

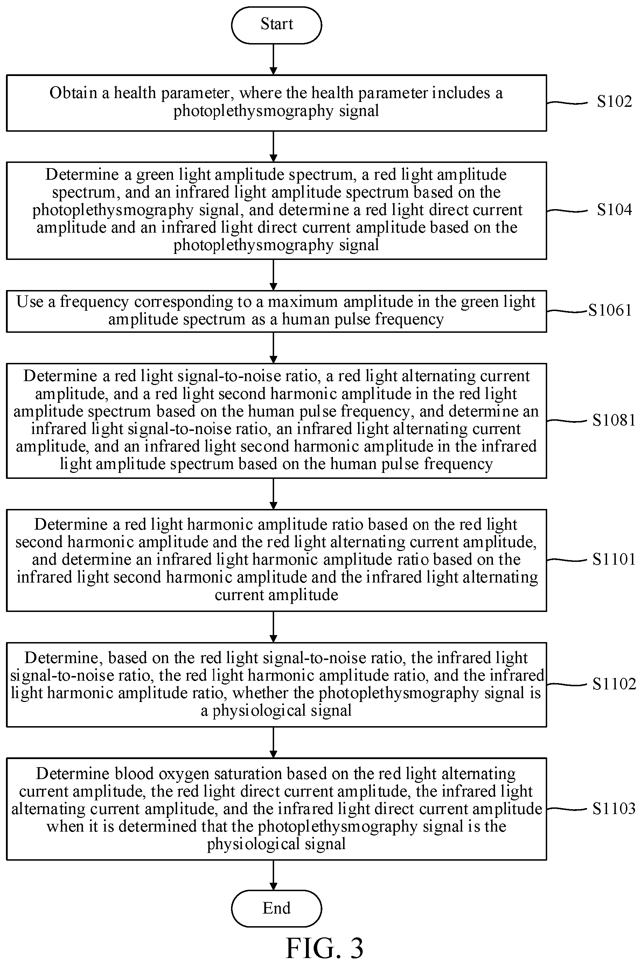

In another embodiment of this application, as shown in FIG. 3, before step S106, the data processing method further includes:
determining a red light direct current amplitude and an infrared light direct current amplitude based on the photoplethysmography signal.

Optionally, this step and the foregoing S104 are the same step. This step is used for determining the red light direct current amplitude (DC_{red}) and the infrared light direct current amplitude (DCᵢᵣ) as parameters for calculating the blood oxygen saturation in subsequent steps. Optionally, a direct current component in the photoplethysmography signal is removed through a second filter, and the green light amplitude spectrum, the red light amplitude spectrum, and the infrared light amplitude spectrum are determined.

Optionally, the second filter is a high-pass filter. The high-pass filter is an electronic filtering apparatus that allows a signal above a cut-off frequency to pass through but blocks a signal below the cut-off frequency. Optionally, the cut-off frequency of the high-pass filter ranges from 0.4 Hz to 0.6 Hz. For example, the cut-off frequency of the high-pass filter is 0.5 Hz. FIG. 8 is a schematic diagram of a photoplethysmography signal with a direct current component filtered out according to an embodiment of this application. After the direct current component in the photoplethysmography signal is removed through the second filter, the green light amplitude spectrum is determined through Fourier transform based on the green light signal; the red light amplitude spectrum is determined through Fourier transform based on the red light signal; and the infrared light amplitude spectrum is determined through Fourier transform based on the infrared light signal. FIG. 9 is a schematic diagram of a green light amplitude spectrum, a red light amplitude spectrum, and an infrared light amplitude spectrum according to an embodiment of this application. It should be noted that the Fourier transform belongs to harmonic analysis. In signal processing, a purpose of the Fourier transform is to decompose a signal into a frequency spectrum (an amplitude spectrum). The frequency spectrum is used to show a magnitude of an amplitude corresponding to a frequency.

S1103 specifically includes: determining the blood oxygen saturation based on the red light alternating current amplitude, the red light direct current amplitude, the infrared light alternating current amplitude, and the infrared light direct current amplitude when it is determined that the photoplethysmography signal is the physiological signal.

Optionally, a first ratio is determined based on the red light alternating current amplitude, the red light direct current amplitude, the infrared light alternating current amplitude, and the infrared light direct current amplitude; and the blood oxygen saturation is determined based on the first ratio.

In another embodiment of this application, as shown in FIG. 3, step S106 includes the following step.

S1061: Use a frequency corresponding to a maximum amplitude in the green light amplitude spectrum as the human pulse frequency.

As shown in FIG. 13, a frequency corresponding to a maximum peak value is found in the green light amplitude spectrum, and the frequency is used as the human pulse frequency. The maximum peak value is the maximum amplitude.

In this embodiment of this application, the human pulse frequency is extracted through green light with a higher signal-to-noise ratio, and the red light signal-to-noise ratio and the infrared light signal-to-noise ratio are determined based on the human pulse frequency. In this approach, signal feature extraction is easier, and the finally calculated blood oxygen saturation can be more accurate.

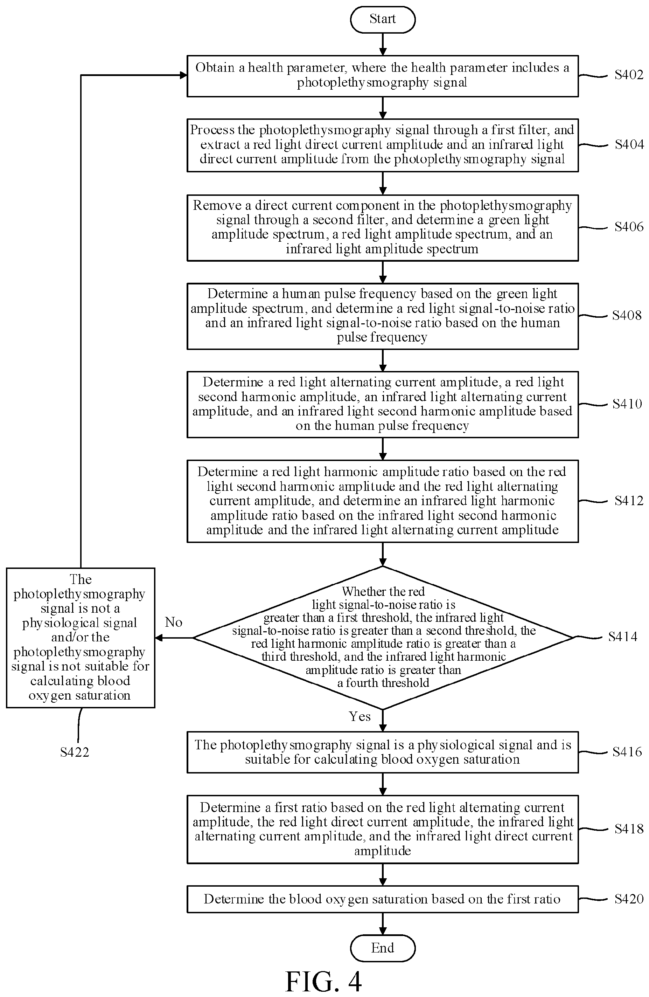

In an embodiment according to this application, as shown in FIG. 4, the data processing method includes the following specific steps.

S402: Obtain a health parameter, where the health parameter includes a photoplethysmography signal.

S404: Process the photoplethysmography signal through a first filter, and extract a red light direct current amplitude and an infrared light direct current amplitude from the photoplethysmography signal.

S406: Remove a direct current component in the photoplethysmography signal through a second filter, and determine the green light amplitude spectrum, the red light amplitude spectrum, and the infrared light amplitude spectrum.

S408: Determine a human pulse frequency based on the green light amplitude spectrum, and determine a red light signal-to-noise ratio and an infrared light signal-to-noise ratio based on the human pulse frequency.

S410: Determine a red light alternating current amplitude, a red light second harmonic amplitude, an infrared light alternating current amplitude, and an infrared light second harmonic amplitude based on the human pulse frequency.

S412: Determine a red light harmonic amplitude ratio based on the red light second harmonic amplitude and the red light alternating current amplitude, and determine an infrared light harmonic amplitude ratio based on the infrared light second harmonic amplitude and the infrared light alternating current amplitude.

S414: Determine whether the red light signal-to-noise ratio is greater than a first threshold, the infrared light signal-to-noise ratio is greater than a second threshold, the red light harmonic amplitude ratio is greater than a third threshold, and the infrared light harmonic amplitude ratio is greater than a fourth threshold, and generate a first determining result.

Four conditions need to be met for determining that the photoplethysmography signal is a physiological signal and suitable for calculating blood oxygen saturation. A first condition is that the red light signal-to-noise ratio is greater than the first threshold; a second condition is that the infrared light signal-to-noise ratio is greater than the second threshold; a third condition is that the red light harmonic amplitude ratio is greater than the third threshold; and a fourth condition is that the infrared light harmonic amplitude ratio is greater than the fourth threshold. When both the red light signal-to-noise ratio and the infrared light signal-to-noise ratio meet a threshold requirement, it indicates that the signal meets a blood oxygen measurement condition. When the red light signal-to-noise ratio or the infrared light signal-to-noise ratio does not meet the threshold requirement, it is considered that the signal does not meet the blood oxygen measurement condition. In addition, because the human photoplethysmography signal generally has a significant second harmonic, and it is usually difficult for interference to form a second harmonic, the presence of a significant second harmonic in a signal spectrum can be used to determine whether the photoplethysmography signal is a physiological signal. Considering that signal strength is affected by factors such as a light emission current of a sensor, when a signal is weak due to a small light emission current, if the signal strength is determined by directly using a magnitude of a second harmonic amplitude, the weak signal is prone to being incorrectly determined as a non-physiological signal. In this case, it is more appropriate to determine, by using a ratio of a second harmonic amplitude to an alternating current amplitude, whether a signal is a physiological signal. When both a signal-to-noise ratio and a harmonic amplitude meet a blood oxygen measurement condition, the blood oxygen saturation is further calculated.

S416: If the first determining result is that the red light signal-to-noise ratio is greater than the first threshold, the infrared light signal-to-noise ratio is greater than the second threshold, the red light harmonic amplitude ratio is greater than the third threshold, and the infrared light harmonic amplitude ratio is greater than the fourth threshold, the photoplethysmography signal is the physiological signal and suitable for calculating the blood oxygen saturation.

When the four conditions (the red light signal-to-noise ratio is greater than the first threshold, the infrared light signal-to-noise ratio is greater than the second threshold, the red light harmonic amplitude ratio is greater than the third threshold, and the infrared light harmonic amplitude ratio is greater than the fourth threshold) are all met, this represents that the photoplethysmography signal is the physiological signal and suitable for calculating the blood oxygen saturation.

S418: Determine a first ratio based on the red light alternating current amplitude, the red light direct current amplitude, the infrared light alternating current amplitude, and the infrared light direct current amplitude.

Optionally, the first ratio is a ratio of a third ratio to a fourth ratio. The third ratio is a ratio of the red light alternating current amplitude to the red light direct current amplitude; and the fourth ratio is a ratio of the infrared light alternating current amplitude to the infrared light direct current amplitude.

S420: Determine the blood oxygen saturation based on the first ratio.

The blood oxygen saturation and the first ratio satisfy a first formula. The first formula is: SₚO₂=A+B×R+C×R². SₚO₂ is the blood oxygen saturation, R is the first ratio, and A, B, and C are constants.

S422: If the first determining result is that the red light signal-to-noise ratio is not greater than the first threshold, the infrared light signal-to-noise ratio is not greater than the second threshold, the red light harmonic amplitude ratio is not greater than the third threshold, or the infrared light harmonic amplitude ratio is not greater than the fourth threshold, the photoplethysmography signal is not the physiological signal and/or the photoplethysmography signal is not suitable for calculating the blood oxygen saturation.

When at least one of the four conditions (the red light signal-to-noise ratio is greater than the first threshold, the infrared light signal-to-noise ratio is greater than the second threshold, the red light harmonic amplitude ratio is greater than the third threshold, and the infrared light harmonic amplitude ratio is greater than the fourth threshold) is not met, this represents that the photoplethysmography signal is not the physiological signal and/or the photoplethysmography signal is not suitable for calculating the blood oxygen saturation, and in this case, returning to S402 is needed.

In this embodiment of this application, the human pulse frequency is determined based on the green light amplitude spectrum, the red light alternating current amplitude is determined based on the red light amplitude spectrum and the human pulse frequency, and the infrared light alternating current amplitude is determined based on the infrared light amplitude spectrum and the human pulse frequency. Compared with an approach to directly extracting the red light alternating current amplitude and the infrared light alternating current amplitude, in this approach to determining the red light alternating current amplitude and the infrared light alternating current amplitude, the obtained red light alternating current amplitude and the obtained infrared light alternating current amplitude are more reliable, and the finally calculated blood oxygen saturation can be more accurate. In addition, compared with an approach to determining quality of the photoplethysmography signal based on a correlation coefficient between an alternating current part in the red light signal and an alternating current part in the green light signal or a correlation coefficient between an alternating current part in the infrared light signal and an alternating current part in the green light signal, an approach to determining, based on a combination of the signal-to-noise ratio and the harmonic amplitude ratio, whether the photoplethysmography signal is a physiological signal and whether the photoplethysmography signal is suitable for calculating the blood oxygen saturation is not prone to incorrectly determining a non-physiological signal as a physiological signal and is not prone to incorrectly determining a weak signal as a non-physiological signal, leading to a more accurate determining result.

As shown in FIG. 5, an embodiment of this application provides a data processing apparatus 500. The data processing apparatus 500 includes an obtaining module 510, a first determining module 520, a second determining module 530, a third determining module 540, and a fourth determining module 550.

The obtaining module 510 is configured to obtain a health parameter, where the health parameter includes a photoplethysmography signal.

The first determining module 520 is configured to determine a green light amplitude spectrum, a red light amplitude spectrum, and an infrared light amplitude spectrum based on the photoplethysmography signal.

The second determining module 530 is configured to determine a human pulse frequency based on the green light amplitude spectrum.

The third determining module 540 is configured to determine a first parameter based on the red light amplitude spectrum and the human pulse frequency, and determine a second parameter based on the infrared light amplitude spectrum and the human pulse frequency.

The fourth determining module 550 is configured to determine blood oxygen saturation based on the first parameter and the second parameter.

Optionally, the third determining module 540 is further configured to determine a red light signal-to-noise ratio, a red light alternating current amplitude, and a red light second harmonic amplitude in the red light amplitude spectrum based on the human pulse frequency, and determine an infrared light signal-to-noise ratio, an infrared light alternating current amplitude, and an infrared light second harmonic amplitude in the infrared light amplitude spectrum based on the human pulse frequency.

Optionally, the fourth determining module 550 is specifically configured to determine a red light harmonic amplitude ratio based on the red light second harmonic amplitude and the red light alternating current amplitude, and determine an infrared light harmonic amplitude ratio based on the infrared light second harmonic amplitude and the infrared light alternating current amplitude; determine, based on the red light signal-to-noise ratio, the infrared light signal-to-noise ratio, the red light harmonic amplitude ratio, and the infrared light harmonic amplitude ratio, whether the photoplethysmography signal is a physiological signal; and determine the blood oxygen saturation when it is determined that the photoplethysmography signal is the physiological signal.

Optionally, the data processing apparatus 500 further includes a fifth determining module 560. The fifth determining module 560 is configured to determine a red light direct current amplitude and an infrared light direct current amplitude based on the photoplethysmography signal.

In addition, the fourth determining module 550 is further configured to determine the blood oxygen saturation based on the red light alternating current amplitude, the red light direct current amplitude, the infrared light alternating current amplitude, and the infrared light direct current amplitude when it is determined that the photoplethysmography signal is the physiological signal.

Optionally, the second determining module 530 is specifically configured to use a frequency corresponding to a maximum amplitude in the green light amplitude spectrum as the human pulse frequency.

In the embodiments of this application, the human pulse frequency is extracted through green light with a higher signal-to-noise ratio (the human pulse frequency is determined based on the green light amplitude spectrum), and the first parameter and the second parameter are determined based on the human pulse frequency. This approach to determining whether conditions for calculating the blood oxygen saturation are met and this approach to determining the parameters used for calculating the blood oxygen saturation lead to easier signal feature extraction and higher reliability of obtained first and second parameters, so that the finally calculated blood oxygen saturation can be more accurate.

The data processing apparatus 500 in this embodiment of this application may be an electronic device, or may be a component, for example, an integrated circuit or a chip, in an electronic device. The electronic device may be a terminal, or may be another device other than a terminal. For example, the electronic device may be a mobile phone, a tablet computer, a notebook computer, a palm computer, an in-vehicle electronic device, a mobile Internet device (Mobile Internet Device, MID), an augmented reality (augmented reality, AR)/virtual reality (virtual reality, VR) device, a robot, a wearable device, an ultra-mobile personal computer (ultra-mobile personal computer, UMPC), a netbook, or a personal digital assistant (personal digital assistant, PDA), or the like, and may alternatively be a server, a network attached storage (Network Attached Storage, NAS), a personal computer (personal computer, PC), a television (television, TV), a teller machine, a self-service machine, or the like. This is not specifically limited in this embodiment of this application.

The data processing apparatus 500 in this embodiment of this application may be an apparatus with an operating system. The operating system may be an Android (Android) operating system, an iOS operating system, or another possible operating system. This is not specifically limited in this embodiment of this application.

The data processing apparatus 500 according to this embodiment of this application can implement all processes implemented by the method embodiments shown in FIG. 1 to FIG. 4, and has beneficial effects of any one of the foregoing embodiments. Details are not described herein again to avoid repetition.

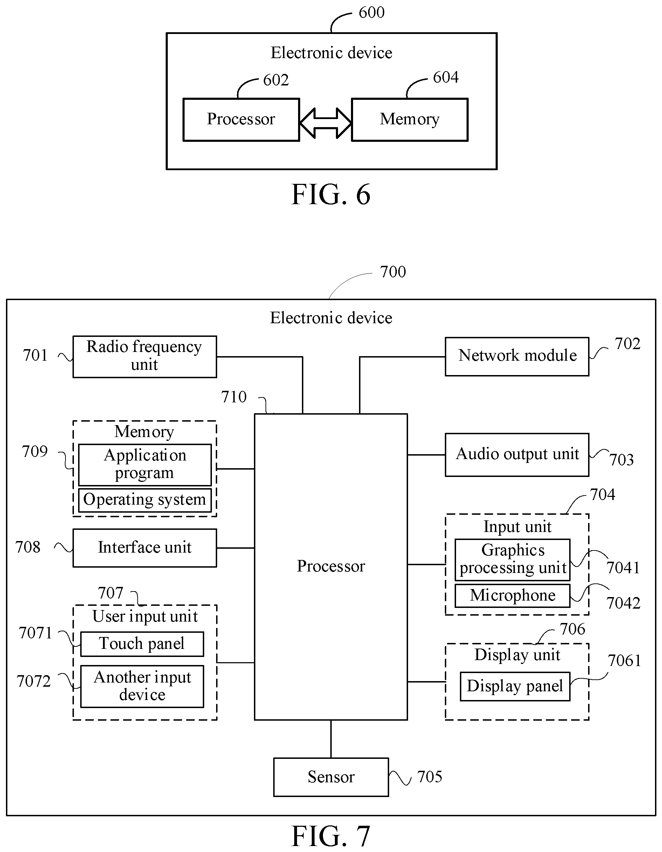

In an embodiment of this application, are shown in FIG. 6, an electronic device 600 includes a processor 602 and a memory 604. The memory 604 stores a program or instructions executable on the processor 602, and when the program or the instructions are executed by the processor 602, each step of the data processing method in any one of the foregoing embodiments is implemented, and the same technical effects can be achieved. Details are not described herein again to avoid repetition.

It should be noted that the electronic device 600 in this embodiment of this application includes a mobile electronic device and a non-mobile electronic device.

FIG. 7 is a schematic structural diagram of hardware of an electronic device according to an embodiment of this application.

The electronic device 700 includes, but is not limited to, components such as a radio frequency unit 701, a network module 702, an audio output unit 703, an input unit 704, a sensor 705, a display unit 706, a user input unit 707, an interface unit 708, a memory 709, and a processor 710.

A person skilled in the art may understand that the electronic device 700 may further include a power supply (such as a battery) for supplying power to the components. The power supply may be logically connected to the processor 710 by using a power management system, thereby implementing functions such as charging, discharging, and power consumption management by using the power management system. The structure of the electronic device shown in FIG. 7 constitutes no limitation on the electronic device, and the electronic device may include more or fewer components than those shown in the figure, or some components may be combined, or a different component deployment may be used, which are not described herein again.

The processor 710 is configured to obtain a health parameter, where the health parameter includes a photoplethysmography signal; determine a green light amplitude spectrum, a red light amplitude spectrum, and an infrared light amplitude spectrum based on the photoplethysmography signal; determine a human pulse frequency based on the green light amplitude spectrum; determine a first parameter based on the red light amplitude spectrum and the human pulse frequency, and determine a second parameter based on the infrared light amplitude spectrum and the human pulse frequency; and determine blood oxygen saturation based on the first parameter and the second parameter.

Optionally, the processor 710 is further configured to determine a red light signal-to-noise ratio, a red light alternating current amplitude, and a red light second harmonic amplitude in the red light amplitude spectrum based on the red light amplitude spectrum and the human pulse frequency, and determine an infrared light signal-to-noise ratio, an infrared light alternating current amplitude, and an infrared light second harmonic amplitude in the infrared light amplitude spectrum and the human pulse frequency based on the human pulse frequency.

Optionally, the processor 710 is specifically configured to determine a red light harmonic amplitude ratio based on the red light second harmonic amplitude and the red light alternating current amplitude, and determine an infrared light harmonic amplitude ratio based on the infrared light second harmonic amplitude and the infrared light alternating current amplitude;

determine, based on the red light signal-to-noise ratio, the infrared light signal-to-noise ratio, the red light harmonic amplitude ratio, and the infrared light harmonic amplitude ratio, whether the photoplethysmography signal is a physiological signal; and
determine the blood oxygen saturation when it is determined that the photoplethysmography signal is the physiological signal.

Optionally, the processor 710 is specifically configured to determine a red light direct current amplitude and an infrared light direct current amplitude based on the photoplethysmography signal; and determine the blood oxygen saturation based on the red light alternating current amplitude, the red light direct current amplitude, the infrared light alternating current amplitude, and the infrared light direct current amplitude when it is determined that the photoplethysmography signal is the physiological signal.

Optionally, the processor 710 is specifically configured to use a frequency corresponding to a maximum amplitude in the green light amplitude spectrum as the human pulse frequency.

It should be understood that, in this embodiment of this application, the input unit 704 may include a graphics processing unit (Graphics Processing Unit, GPU) 7041 and a microphone 7042, and the graphics processing unit 7041 processes static pictures or video image data obtained by an image capturing device (such as a camera) in a video capturing mode or an image capturing mode. The display unit 706 may include a display panel 7061, and the display panel 7061 may be configured by using a liquid crystal display, an organic light-emitting diode, or the like. The user input unit 707 includes at least one of a touch panel 7071 and another input device 7072. The touch panel 7071 is also referred to as a touch screen. The touch panel 7071 may include two parts: a touch detection apparatus and a touch controller. The another input device 7072 may include, but is not limited to, a physical keyboard, a functional key (such as a volume control key or a switch key), a track ball, a mouse, and a joystick, which are not described herein again.

The memory 709 may be configured to store a software program and various data. The memory 709 may mainly include a first storage area storing a program or instructions and a second storage area storing data, where the first storage area may store an operating system, an application program or instructions required by at least one function (such as a sound playing function or an image playing function), and the like. In addition, the memory 709 may include a volatile memory or a non-volatile memory. Alternatively, the memory 709 may include both a volatile memory and a non-volatile memory.

The non-volatile memory may be a read-only memory (Read-Only Memory, ROM), a programmable ROM (Programmable ROM, PROM), an erasable PROM (Erasable PROM, EPROM), an electrically EPROM (Electrically EPROM, EEPROM), or a flash memory. The volatile memory may be a random access memory (Random Access Memory, RAM), a static RAM (Static RAM, SRAM), a dynamic RAM (Dynamic RAM, DRAM), a synchronous DRAM (Synchronous DRAM, SDRAM), a double data rate SDRAM (Double Data Rate SDRAM, DDRSDRAM), an enhanced SDRAM (Enhanced SDRAM, ESDRAM), a synch link DRAM (Synch link DRAM, SLDRAM), or a direct rambus RAM (Direct Rambus RAM, DRRAM). The memory 709 in this embodiment of this application includes, but is not limited to, these memories and any other suitable types of memories.

Optionally, the processor 710 may comprise one or more processing units. Optionally, the processor 710 integrates an application processor and a modem processor, where the application processor mainly processes an operation involving the operating system, the user interface, the application program, and the like, and the modem processor mainly processes a wireless communication signal, for example, a baseband processor. It may be understood that the foregoing modem processor may alternatively not be integrated into the processor 710.

In an embodiment of this application, a readable storage medium stores a program or instructions, where when the program or the instructions are executed by a processor, each process of the data processing method in any one of the foregoing embodiments is implemented and the same technical effects can be achieved. Details are not described herein again to avoid repetition.

The processor is the processor in the electronic device in the foregoing embodiment. The readable storage medium includes a computer-readable storage medium, for example, a computer read-only memory ROM, a random access memory RAM, a magnetic disk, or an optical disk.

In an embodiment of this application, a chip includes a processor and a communication interface, where the communication interface is coupled to the processor, and the processor is configured to execute a program or instructions to implement each process of the data processing method in any one of the foregoing embodiments and the same technical effects can be achieved. Details are not described herein again to avoid repetition.

It should be understood that the chip mentioned in this embodiment of this application may also be referred to as a system-level chip, a system chip, a chip system, a system-on-a-chip chip, or the like.

In an embodiment of this application, a computer program product is stored in a storage medium, and the program product is executed by at least one processor to implement each process of the data processing method in any one of the foregoing embodiments, and the same technical effects can be achieved. Details are not described herein again to avoid repetition.

It should be noted that the term "comprise", "include" or any other variation thereof in this specification is intended to cover a non-exclusive inclusion, which specifies the presence of stated processes, methods, objects, or apparatuses, but does not preclude the presence or addition of one or more other processes, methods, objects, or apparatuses. Without further limitations, elements defined by the sentence "including one" does not exclude that there are still other same elements in the process, method, object, or apparatus.

In addition, it should be noted that, the scope of the method and apparatus in the embodiments of this application is not limited to performing the functions in the order shown or discussed, but may also include performing the functions in a substantially simultaneous manner or in a reverse order according to the functions involved, for example, the described method may be performed in a sequence different from the described order, and various steps may also be added, omitted, or combined. In addition, features described with reference to certain examples may be combined in other examples.

Through the description of the foregoing implementations, a person skilled in the art may clearly understand that the method according to the foregoing embodiments may be implemented by means of software and a necessary general hardware platform, and certainly, may also be implemented by hardware, but in many cases, the former manner is a better implementation. Based on such an understanding, the technical solutions of this application essentially or the part contributing to the related art may be implemented in a form of a computer software product. The computer software product is stored in a storage medium (such as a ROM/RAM, a magnetic disk, or an optical disk) and includes several instructions for instructing a terminal (which may be a mobile phone, a computer, a server, a network device, or the like) to perform the methods in the embodiments of this application.

The embodiments of this application are described above with reference to the accompanying drawings, but this application is not limited to the foregoing specific embodiments, which are merely illustrative rather than limited. Under the inspiration of this application, a person of ordinary skill in the art can make many forms without departing from the scope of this application and the protection of the claims, all of which fall within the protection of this application.

## Claims

1. A data processing method, comprising:
obtaining a health parameter, wherein the health parameter comprises a photoplethysmography signal;
determining a green light amplitude spectrum, a red light amplitude spectrum, and an infrared light amplitude spectrum based on the photoplethysmography signal;
determining a human pulse frequency based on the green light amplitude spectrum;
determining a first parameter based on the red light amplitude spectrum and the human pulse frequency, and determining a second parameter based on the infrared light amplitude spectrum and the human pulse frequency; and
determining blood oxygen saturation based on the first parameter and the second parameter.

2. The data processing method according to claim 1, wherein the determining a first parameter based on the red light amplitude spectrum and the human pulse frequency, and determining a second parameter based on the infrared light amplitude spectrum and the human pulse frequency comprises:
determining a red light signal-to-noise ratio, a red light alternating current amplitude, and a red light second harmonic amplitude in the red light amplitude spectrum based on the human pulse frequency, and determining an infrared light signal-to-noise ratio, an infrared light alternating current amplitude, and an infrared light second harmonic amplitude in the infrared light amplitude spectrum based on the human pulse frequency.

3. The data processing method according to claim 2, wherein the determining blood oxygen saturation based on the first parameter and the second parameter comprises:
determining a red light harmonic amplitude ratio based on the red light second harmonic amplitude and the red light alternating current amplitude, and determining an infrared light harmonic amplitude ratio based on the infrared light second harmonic amplitude and the infrared light alternating current amplitude;
determining, based on the red light signal-to-noise ratio, the infrared light signal-to-noise ratio, the red light harmonic amplitude ratio, and the infrared light harmonic amplitude ratio, whether the photoplethysmography signal is a physiological signal; and
determining the blood oxygen saturation when it is determined that the photoplethysmography signal is the physiological signal.

4. The data processing method according to claim 3, wherein before the determining a human pulse frequency based on the green light amplitude spectrum, the data processing method further comprises:
determining a red light direct current amplitude and an infrared light direct current amplitude based on the photoplethysmography signal; and
the determining the blood oxygen saturation when it is determined that the photoplethysmography signal is the physiological signal comprises:
determining the blood oxygen saturation based on the red light alternating current amplitude, the red light direct current amplitude, the infrared light alternating current amplitude, and the infrared light direct current amplitude when it is determined that the photoplethysmography signal is the physiological signal.

5. The data processing method according to any one of claims 1 to 4, wherein the determining a human pulse frequency based on the green light amplitude spectrum comprises:
using a frequency corresponding to a maximum amplitude in the green light amplitude spectrum as the human pulse frequency.

6. A data processing apparatus, comprising:
an obtaining module, configured to obtain a health parameter, wherein the health parameter comprises a photoplethysmography signal;
a first determining module, configured to determine a green light amplitude spectrum, a red light amplitude spectrum, and an infrared light amplitude spectrum based on the photoplethysmography signal;
a second determining module, configured to determine a human pulse frequency based on the green light amplitude spectrum;
a third determining module, configured to determine a first parameter based on the red light amplitude spectrum and the human pulse frequency, and determine a second parameter based on the infrared light amplitude spectrum and the human pulse frequency; and
a fourth determining module, configured to determine blood oxygen saturation based on the first parameter and the second parameter.

7. The data processing apparatus according to claim 6, wherein the third determining module is further configured to determine a red light signal-to-noise ratio, a red light alternating current amplitude, and a red light second harmonic amplitude in the red light amplitude spectrum based on the human pulse frequency, and determine an infrared light signal-to-noise ratio, an infrared light alternating current amplitude, and an infrared light second harmonic amplitude in the infrared light amplitude spectrum based on the human pulse frequency.

8. The data processing apparatus according to claim 7, wherein the fourth determining module is specifically configured to determine a red light harmonic amplitude ratio based on the red light second harmonic amplitude and the red light alternating current amplitude, and determine an infrared light harmonic amplitude ratio based on the infrared light second harmonic amplitude and the infrared light alternating current amplitude;
determine, based on the red light signal-to-noise ratio, the infrared light signal-to-noise ratio, the red light harmonic amplitude ratio, and the infrared light harmonic amplitude ratio, whether the photoplethysmography signal is a physiological signal; and
determine the blood oxygen saturation when it is determined that the photoplethysmography signal is the physiological signal.

9. The data processing apparatus according to claim 8, further comprising:
a fifth determining module, configured to determine a red light direct current amplitude and an infrared light direct current amplitude based on the photoplethysmography signal, wherein
the fourth determining module is further configured to determine the blood oxygen saturation based on the red light alternating current amplitude, the red light direct current amplitude, the infrared light alternating current amplitude, and the infrared light direct current amplitude when it is determined that the photoplethysmography signal is the physiological signal.

10. The data processing apparatus according to any one of claims 6 to 9, wherein the second determining module is specifically configured to use a frequency corresponding to a maximum amplitude in the green light amplitude spectrum as the human pulse frequency.

11. An electronic device, comprising a processor and a memory, wherein the memory stores a program or instructions executable on the processor, and when the program or the instructions are executed by the processor, the steps of the data processing method according to any one of claims 1 to 5 are implemented.

12. A readable storage medium, storing a program or instructions, wherein when the program or the instructions are executed by a processor, the steps of the data processing method according to any one of claims 1 to 5 are implemented.

13. A computer program product, stored in a storage medium, wherein the program product is executed by at least one processor to implement the steps of the data processing method according to any one of claims 1 to 5.

14. An electronic device, configured to perform the steps of the data processing method according to any one of claims 1 to 5.

15. A chip, comprising a processor and a communication interface, wherein the communication interface is coupled to the processor, and the processor is configured to execute a program or instructions to implement the steps of the data processing method according to any one of claims 1 to 5.
